# EUROPEAN PATENT APPLICATION

(11) **EP 4 516 305 A1**
(43) Date of publication of application: **05.03.2025**
(21) Application number: 23796397.0
(22) Date of filing: 25.04.2023
(51) Int. Cl.: A61K 35/747, A23L 33/18, A61K 31/343, A61K 31/366, A61K 31/44, A61K 31/4439, A61K 31/496, A61K 31/69, A61K 31/7088, A61K 38/02, A61K 38/16, A61K 38/20, A61K 45/00, A61K 48/00, A61P 27/10, C07K 14/54

(54) **METHOD AND COMPOSITION FOR PREVENTING OR TREATING MYOPIA**

(30) Priority: 26.04.2022 JP 2022072596
(71) Applicant: Tsubota Laboratory, Inc., Tokyo 160-0016 (JP)
(72) Inventor: TSUBOTA, Kazuo, Tokyo 160-0016 (JP); KURIHARA, Toshihide, Tokyo 160-0016 (JP); IKEDA, Shinichi, Tokyo 160-0016 (JP); HOU, Jing, Tokyo 160-0016 (JP)
(74) Representative: Plasseraud IP
(86) International application number: PCT/JP2023/016344
(87) International publication number: WO 2023/210657

(57) **Abstract**

An object of the present invention is to provide a method for preventing or treating myopia, and a composition used in said method. The present invention provides a method and composition for inhibiting, improving, or treating myopia by administering a substance that induces M2 macrophage polarization, a substance that regulates the function of choroid-resident immune cells, such as a mast cell stabilizer or a chemical mediator release inhibitor, or lactic acid bacteria. Regulating the function of choroid-resident immune cells, for example by inducing the polarization of M2 macrophages in the choroid or by administering a mast cell stabilizer, a chemical mediator release inhibitor, or lactic acid bacteria, is effective in myopia prevention and treatment.

## Description

### Technical Field

The present invention generally relates to a method and a composition for preventing or treating myopia, more specifically to a composition that acts on immune cells in the choroid to inhibit progression of myopia, and more specifically to a method and a composition for inducing M2 macrophage polarization or regulating the function of choroid-resident immune cells, such as administrating a mast cell stabilizer, a chemical mediator release inhibitor, or lactic acid bacteria.

### Background Art

Myopia refers to a condition in which focus is formed in front of the retina due to axial elongation of the ocular globe, and myopia becomes more severe as the ocular elongation increases. With the increase in prevalence of myopia, there is an increased interest in factors involved in myopia pathogenesis and methods for preventing and suppressing the myopia progression. Recent studies have suggested that the choroid is important in regulation of eye growth and development of myopia, demonstrating that choroidal thinning is a structural feature of myopia. With a negative correlation between choroidal thickness and axial length, it is suggested that change in choroidal thickness may be a predictive biomarker for ocular elongation. However, the detailed mechanism by which the choroid is involved in the development and progression of myopia remains unknown.

The choroid is a tissue that covers the outer side of the retina, which is rich in small blood vessels, and the choroid has, in addition to a function to supply oxygen and nutrients to retina cells, a function to supply growth factors that are involved in regulation of eye growth and tissue remodeling of the sclera on the outside of the ocular globe. It is thought that decrease in choroidal thickness or in blood flow contributes to scleral ischemia and hypoxia, which is thought to affect changes in the scleral structure resulting in ocular elongation. Therefore, maintaining and increasing the choroidal thickness and blood flow has attracted attention as a new target in prevention and treatment of myopia.

Methods for maintaining and increasing the choroidal thickness to suppress the progression of myopia have been proposed, such as dietary supplementation of crocetin (Non-Patent Document 1) and irradiation of violet light (Non-Patent Document 2).

Crocetin is known to have anti-inflammatory and immunomodulatory effects, and is known to express the effects through the adjustment of the balance between T cells Th1/Th2 and Th17/Treg, and inhibition of the NF-κB pathway of cells that produce inflammation mediators (such as TNF-α, IL-6, and IFN-γ) such as macrophages (Non-Patent Documents 3-5).

Thirteen types of cells exist in the choroid, of which four are immune cells. Tissue-resident immune cells are responsible for maintaining tissue homeostasis and structure, in addition to immune responses, and, for example, it has been reported that choroidal thinning occurs in mice lacking macrophages or in mice with induced mast cell degranulation (Non-Patent Documents 6-7).

### Prior Art Document

### Non-Patent Literature

[Non-Patent Document 1] Mori et al., Sci Rep. 2019. 22;9(1): 295.
[Non-Patent Document 2] Jiang et al. Proc Natl Acad Sci USA. 2021. 1;118(22):e2018840118.
[Non-Patent Document 3] Oxid Med Cell Longev. 2021 Sep 10;2021:6631929.
[Non-Patent Document 4] Eur J Pharmacol. 2012 Jan 15;674(2-3):391-6.
[Non-Patent Document 5] Biofactors. 2023 Feb 6. doi: 10.1002/biof.1942.
[Non-Patent Document 6] Elife. 2020 Apr 1;9:e55564.
[Non-Patent Document 7] FASEB J. 2020 Aug;34(8):10117-10131.

### Summary of the Invention

### Problem To Be Solved by the Invention

An object of the present disclosure is to provide a method for preventing or treating myopia, a composition used in said method, in particular a composition targeting choroid-resident immune cells, particularly macrophages and mast cells.

### Means To Solve the Problem

The present inventors found that M2 macrophage polarization of macrophages in the choroid suppresses choroidal thinning and is effective in preventing and treating myopia.

In addition, mast cells are distributed around small blood vessels in large numbers and are also found in the choroid, which is a tissue composed of blood vessels, and are thus suggested to be involved in maintaining the morphology of the choroid. The present inventors found that progression of myopia is suppressed by ocular instillation of a drug (mast cell stabilizer) that inhibits mast cell degranulation. That is, the present inventors found that ocular instillation of a mast cell stabilizer (cromolyn sodium, pemirolast potassium), which is a type of anti-allergic drug, to a minus-lens-induced myopia mouse model results in suppression of the progression of myopia. It should be noted that progression of myopia was not suppressed with ocular instillation of an antihistamine (levocabastine), which is another anti-allergic drug, to mice undergoing myopia induction by fitting a minus lens.

Furthermore, it is known that administration of lactic acid bacteria of the genus Lactobacillus, such as Lactobacillus paracasei, induces and activates M2 macrophage polarization, and the present inventors found that the administration of lactic acid bacteria of the genus Lactobacillus, such as Lactobacillus paracasei suppresses myopia progression. In consideration of the foregoing and the findings of Non-Patent Documents 6-7 described above, it can be considered that, if the choroid structure (thickness) can be maintained as a result of intervention targeting choroid-resident immune cells, it would have an inhibitory effect against myopia progression. Based on these findings, it is thought that a composition that targets immune cells present in the choroid, such as macrophages and mast cells, and that guides the properties thereof to appropriate states would have an inhibitory effect on myopia progression. The present invention is based on such findings and includes the following embodiments.

### [Embodiment 1]

A method for inhibiting, improving, or treating myopia, comprising administering to a subject in need thereof a therapeutically effective amount of a choroid-resident immune cell function regulator or lactic acid bacteria.

### [Embodiment 2]

The method according to Embodiment 1, wherein the choroid-resident immune cell function regulator is an M2 macrophage polarization inducer, a mast cell stabilizer, or a chemical mediator release inhibitor.

### [Embodiment 3]

The method according to Embodiment 2, wherein the M2 macrophage polarization inducer is selected from the group consisting of: proteins, peptides, nucleic acids, small molecule compounds, and polymeric compounds.

### [Embodiment 4]

The method according to Embodiment 2, wherein the M2 macrophage polarization inducer is selected from the group consisting of: IL-4, IL-10, IL-13, TLR2, 4, 7, 9 and ligands thereof, bisantrene dihydrochloride, triptolide, lovastatin, QS11, regorafenib, sorafenib, ixazomib, GW-843682X, KW 2449, axitinib, JTE 013, purmorphamine, arcyriaflavin A, dasatinib, NVP-LDE225, 1-naphthyl PP1, MGCD-265, and bosutinib.

### [Embodiment 5]

The method according to Embodiment 2, wherein the mast cell stabilizer or the chemical mediator release inhibitor is cromolyn, pemirolast or a salt thereof.

### [Embodiment 6]

The method according to Embodiment 1, wherein the lactic acid bacteria is of the genus Lactobacillus.

### [Embodiment 7]

The method according to Embodiment 1, wherein the lactic acid bacteria is Lactobacillus paracasei.

### [Embodiment 8]

The method according to Embodiment 1, wherein the choroid-resident immune cell function regulator or the lactic acid bacteria is contained in a pharmaceutical composition, a supplement, or a food product.

### [Embodiment 9]

The method according to Embodiment 1, wherein refractive loss, ocular elongation, and/or choroidal thinning is inhibited.

### [Embodiment 10]

A composition for inhibiting or treating myopia, the composition comprising a therapeutically effective amount of a choroid-resident immune cell function regulator or lactic acid bacteria.

### [Embodiment 11]

A supplement for inhibiting or improving myopia, the supplement comprising an effective amount of a choroid-resident immune cell function regulator or lactic acid bacteria.

### [Embodiment 12]

A food product for inhibiting or improving myopia, the food product comprising an effective amount of a choroid-resident immune cell function regulator or lactic acid bacteria.

### [Embodiment 13]

Use of a choroid-resident immune cell function regulator or lactic acid bacteria in manufacture of a pharmaceutical for inhibiting or treating myopia.

### [Embodiment 14]

Use of a choroid-resident immune cell function regulator or lactic acid bacteria for inhibiting or treating myopia.

### Effects of the Invention

According to the present disclosure, it is possible to provide a novel method of preventing or treating myopia by regulating the function of choroid-resident immune cells, and, more specifically, inducing M2 macrophage polarization in the choroid, or administering a mast cell stabilizer, a chemical mediator release inhibitor, or lactic acid bacteria.

### Brief Description of the Drawings

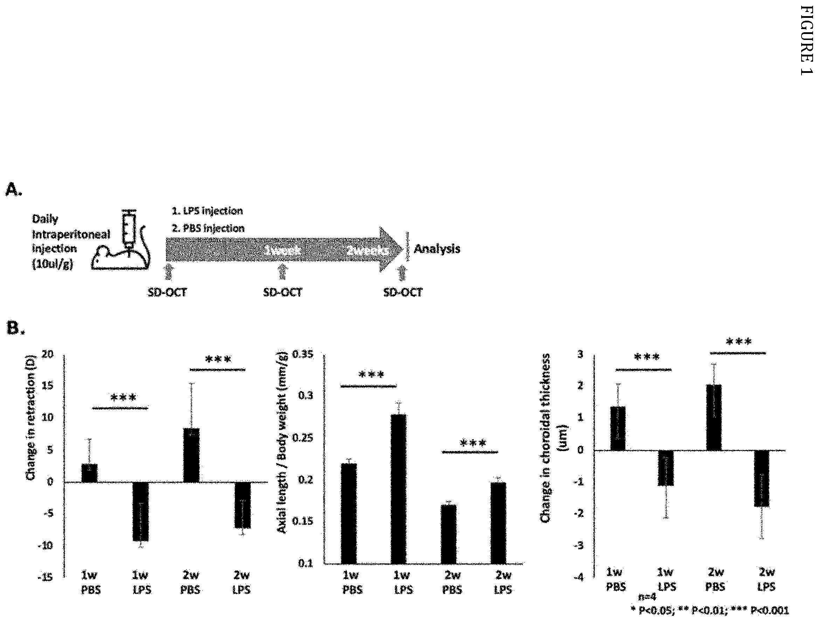

[Figure 1] shows results of myopia induction by means of LPS administration. (A) is a diagram showing the schedule of experiment. (B) shows graphs indicating changes in refractive value (left), axial length (center), and choroidal thickness (right).

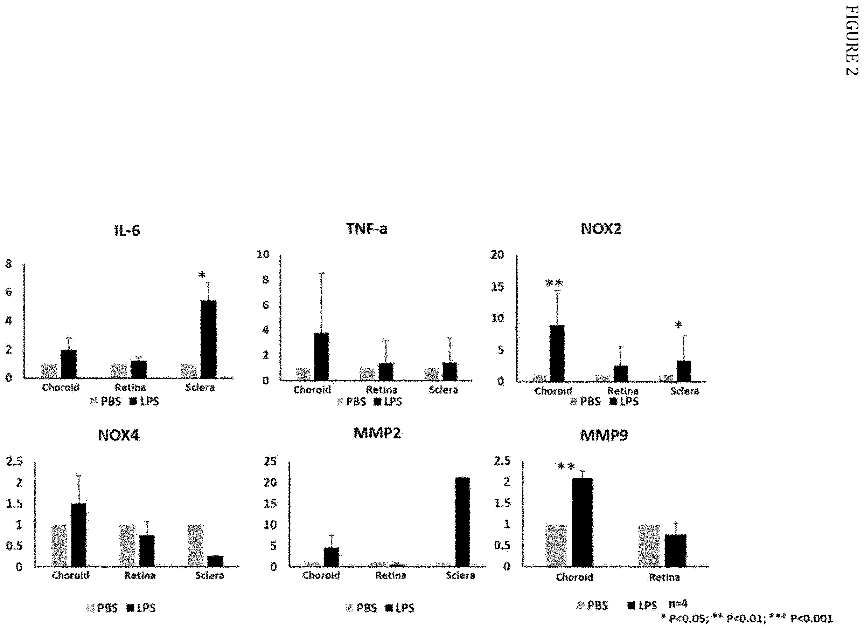

[Figure 2] shows graphs indicating changes in expression of M1 macrophage marker genes and oxidative-stress-related genes due to LPS administration.

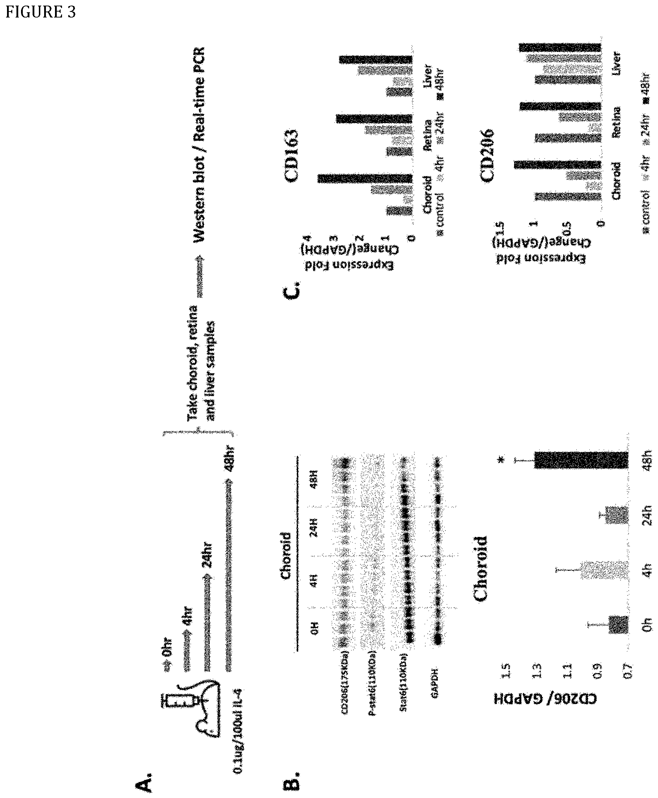

[Figure 3] shows results of M2 macrophage polarization induction by means of IL-4 administration. (A) is a diagram showing the schedule of experiment. (B) is a graph indicating changes in expression of CD206 protein and phosphorylated STAT6 in the choroid due to IL-4 administration. (C) shows graphs indicating changes in expression of CD163 and CD206 mRNA due to IL-4 administration.

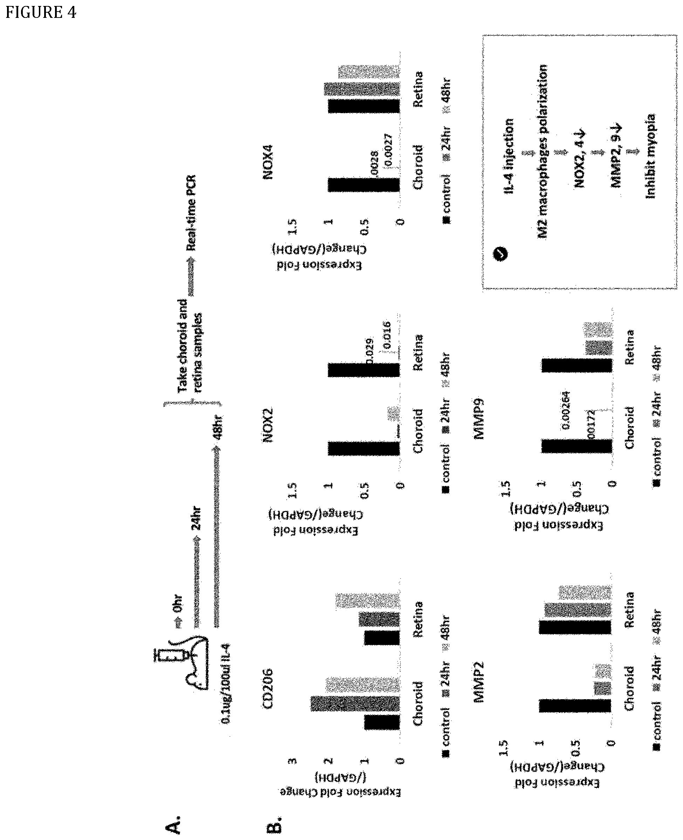

[Figure 4] shows graphs indicating changes in expression of M2 macrophage marker genes and oxidative-stress-related genes due to IL-4 administration.

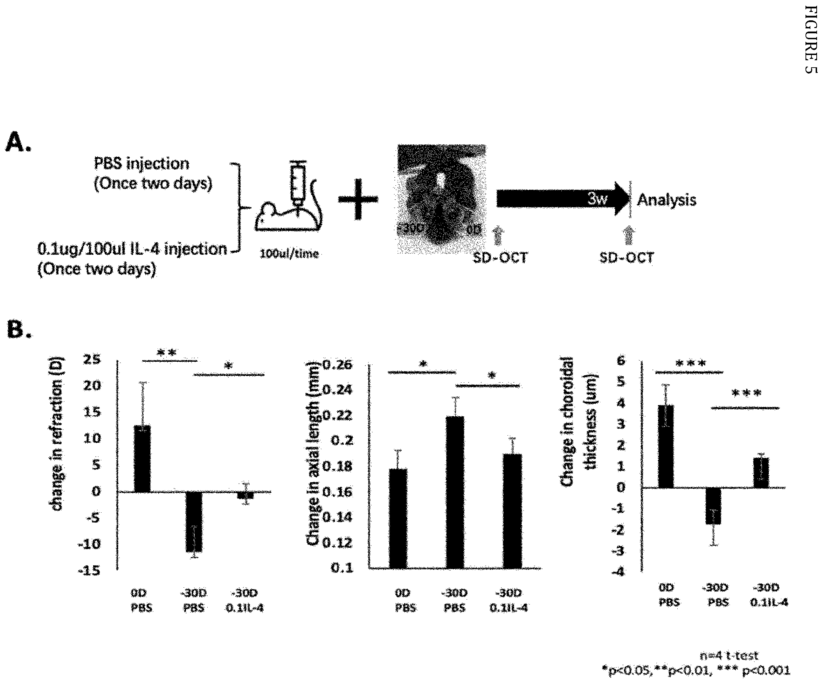

[Figure 5] shows results of an experiment to suppress myopization by means of IL-4 administration. (A) is a diagram showing the schedule of experiment. (B) shows diagrams representing changes in refraction (left), axial length (center), and choroidal thickness (right), due to IL-4 administration during the myopia induction period.

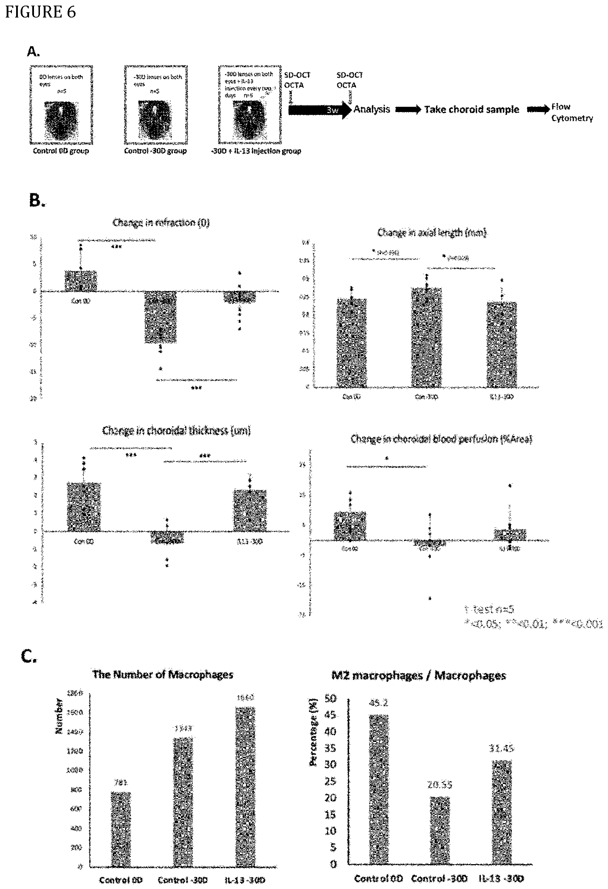

[Figure 6] shows results of suppression of myopization and M2 macrophage polarization induction by means of IL-13 administration. (A) is a diagram showing the schedule of experiment. (B) shows graphs representing changes in refraction (upper left), axial length (upper right), choroidal thickness (lower left), and blood flow (lower right), due to IL-13 administration during the myopia induction period. (C) shows graphs representing changes in the number of macrophages and the M2 macrophage ratio due to myopia induction and IL-13 administration.

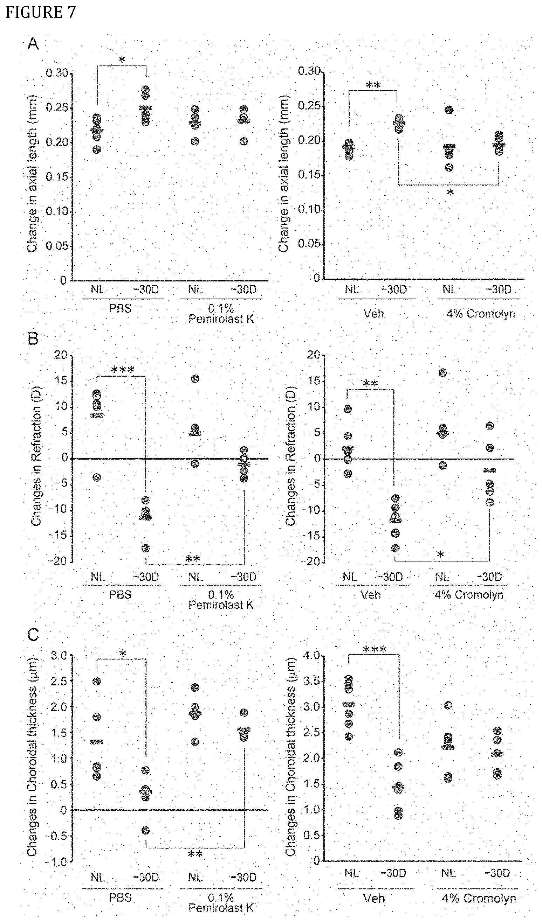

[Figure 7] shows results of the myopia suppression effect by means of ocular instillation of mast cell stabilizers. (A) is a graph showing the amount of change in axial length. (B) is a graph showing the amount of change in refractive value. (C) is a graph showing the amount of change in choroidal thickness. In each of the above, the left graph shows the control group vs. pemirolast potassium instillation group, and the right graph shows the control group vs. cromolyn instillation group. *p<0.05, **p<0.01, ***p<0.001, student's t-test

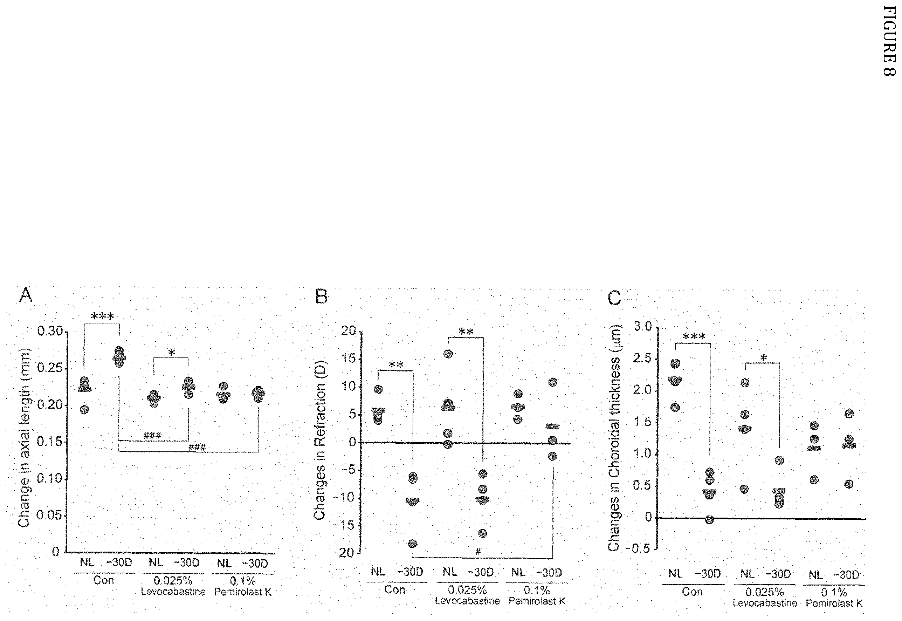

[Figure 8] shows graphs representing a comparison of the myopia suppression effect of a mast cell stabilizer eye drop and a histamine receptor antagonist. (A) is a graph showing the amount of change in axial length. (B) is a graph showing the amount of change in refractive value. (C) is a graph showing the amount of change in choroidal thickness. The respective graphs show, in order from the left, the control group, the levocabastine instillation group (histamine receptor antagonist), and the pemirolast potassium instillation group (mast cell stabilizer). *p<0.05, **p<0.01, ***p<0.001, student's t-test

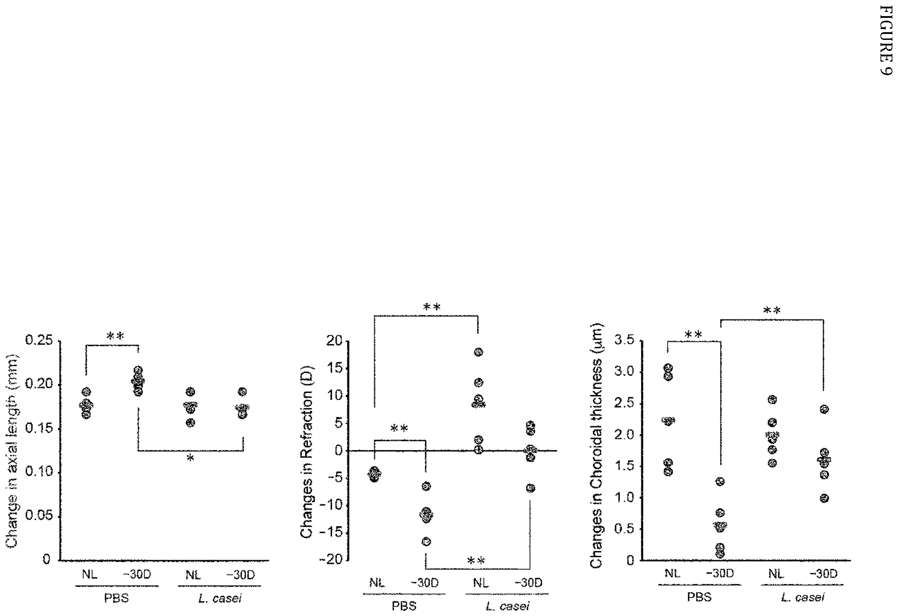

[Figure 9] shows results of the myopia progression suppression effect by means of administration of Lactobacillus paracasei. (A) is a graph showing the amount of change in axial length. (B) is a graph showing the amount of change in refractive value. (C) is a graph showing the amount of change in choroidal thickness. In each of the above, the left graph shows the control group and the right graph shows the Lactobacillus paracasei administration group.

### Embodiments To Carry Out the Invention

Details of the present invention will be described. The present invention is not limited to the content of the following embodiments and examples, and includes various modified examples and application examples within the scope of the gist of the present invention.

### [Method for inhibiting or treating myopia]

As described above, it has become clear in recent years that change in the choroid known as thinning is involved in the development of myopia. As a result of extensive research into factors that control this thinning, the present inventors found that changes (polarization) in the state of macrophages in the choroid cause changes in choroidal thickness, thereby controlling myopia progression.

Macrophage states include M1 macrophages and M2 macrophages, in which M1 macrophages are mainly involved in the induction of inflammation, and M2 macrophages play an antagonistic role, involved in the resolution and suppression of inflammation. The present inventors found, for the first time, that inducing M1 macrophage polarization in the choroid leads to choroidal thinning and myopia progression, whereas inducing M2 macrophage polarization suppresses choroidal thinning and myopia progression. In addition, mast cells are distributed around small blood vessels in large numbers and are also found in the choroid, which is a tissue composed of blood vessels, and are thus suggested to be involved in maintaining the morphology of the choroid. The present inventors found that progression of myopia is suppressed by ocular instillation of a drug (mast cell stabilizer) that inhibits mast cell degranulation. Furthermore, it is known that administration of lactic acid bacteria of the genus Lactobacillus, such as Lactobacillus paracasei, induces and activates M2 macrophage polarization, and the present inventors found that the administration of lactic acid bacteria of the genus Lactobacillus, such as Lactobacillus paracasei suppresses myopia progression. In this manner, the present inventors found that it is possible to suppress myopia progression by regulating the function of choroid-resident immune cells.

Accordingly, one aspect of the present disclosure relates to a method of suppressing, improving, or treating myopia by regulating the function of choroid-resident immune cells, and, more specifically, by causing M2 macrophage polarization of macrophages in the choroid, or by administering a mast cell stabilizer, a chemical mediator release inhibitor, or lactic acid bacteria.

One aspect of the present disclosure relates to a method of suppressing, improving, or treating myopia by regulating the function of choroid-resident immune cells, and, more specifically, inducing M2 macrophage polarization in the choroid using an M2 macrophage polarization inducer, or administering a mast cell stabilizer, a chemical mediator release inhibitor, or lactic acid bacteria.

One aspect of the present disclosure relates to a method of inhibiting refractive loss, ocular elongation, and/or choroidal thinning, comprising regulating the function of choroid-resident immune cells, and, more specifically, delivering an M2 macrophage polarization inducer to a subject's choroid, or administering a mast cell stabilizer, a chemical mediator release inhibitor, or lactic acid bacteria, in a subject in need of treatment.

One aspect of the present disclosure also relates to a screening method for searching for a substance that regulates the function of choroid-resident immune cells, such as a component that promotes M2 macrophage polarization, in order to control choroidal thinning, which is the mechanism of myopia pathogenesis, and a screening method for searching for a component that is effective in the prevention and treatment of myopia.

### <Choroid-resident immune cell function regulator>

A choroid-resident immune cell function regulator is a substance that regulates the function of choroid-resident immune cells. Preferred examples of substances that regulate the function of choroid-resident immune cells include substances that perform regulation for inhibiting inflammation in the choroid and substances that regulate the choroid to be an anti-inflammatory environment. Examples of choroid-resident immune cells include macrophages and mast cells. Examples of choroid-resident immune cell function regulators include M2 macrophage polarization inducers, mast cell stabilizers, chemical mediator release inhibitors, and lactic acid bacteria.

### <M2 macrophage polarization inducer>

Macrophages are classified into functionally different M1 macrophages, which are inflammatory, and M2 macrophages, which are anti-inflammatory; the macrophages are polarized in response to signals from cytokines and stimulatory components to express specific functions.

Therefore, the M2 macrophage polarization inducer in the present disclosure includes various substances that are involved in the transmission of signals for inducing M2 macrophage polarization in the choroid, and proteins, peptides, nucleic acid drugs, small molecule compounds, polymeric compounds, and the like, may be used without particular limitation.

Examples of such substances include cytokines produced by Th2 cells, such as IL-4 and IL-3, as well as the leucine zipper transcription factor c-Maf and the carbohydrate-binding lectin galectin-3. In addition, it is known that there are several populations of M2 macrophages, so, in addition to the above-mentioned IL-4 and IL-13, which induce M2a macrophages, IL-10, which is involved in M2c macrophage induction, glucocorticoid hormones (GC), TLR2, TRL4, TRL7, TLR9 and ligands thereof (ligands for TLRs), which are involved in M2d macrophage induction, and the like, are also examples of M2 macrophage polarization inducers in the present disclosure.

Examples of M2 macrophage polarization inducers in the present disclosure include various small molecule compounds, such as bisantrene dihydrochloride, triptolide, lovastatin, QS11, regorafenib, sorafenib, ixazomib, GW-843682X, KW 2449, axitinib, JTE 013, purmorphamine, arcyriaflavin A, dasatinib, NVP-LDE225, 1-naphthyl PP1, MGCD-265, and bosutinib.

Agonists of the substances described above may also be used as M2 macrophage polarization inducers. It should be noted that a person skilled in the art could, for example, culture cells such as monocytic cell lines, e.g., RAW264 cells, J774 cells, and U937 cells, mouse peritoneal macrophages, and bone marrow-derived macrophages, in a medium containing the substance to be evaluated, and use an in vitro evaluation system, etc., for evaluating the expression of M2 macrophage markers to evaluate whether the substance has the ability to induce M2 macrophage polarization, to obtain an M2 macrophage polarization inducer suitable for use in the method and composition of the present disclosure, other than the M2 macrophage polarization inducers specifically recited in the present Specification.

When the M2 macrophage polarization inducer is a protein or a peptide, the substance can be administered to the subject in the form of DNA or RNA that encodes the protein or peptide. A nucleic acid that encodes the M2 macrophage polarization inducer may be administered to the subject using a plasmid or expression vector. The expression vector may be, but is not limited to, a viral vector, particularly an adenoviral vector. Other viral vectors that can be used include retrovirus, adeno-associated virus, pox, baculovirus, vaccinia, herpes simplex, Epstein-Barr, geminivirus, and caulimovirus vectors. A nucleic acid that encodes an M2 macrophage polarization inducer may contain a regulatory element for choroid/RPE-specific expression of the protein. That is, the nucleic acid that encodes the M2 macrophage polarization inducer may be operably linked to a regulatory element, such as a promoter or an enhancer.

### <Mast cell stabilizer>

Mast cell stabilizers are also called mast cell stabilizing agents, mast cell stabilizing drugs, and the like. A mast cell stabilizer is, for example, a drug that suppresses the release of allergens from mast cells, such as a drug that stabilizes the cell membrane of mast cells to suppress the release of allergens from the mast cells. Examples of mast cell stabilizers include acitazanolast hydrate solution (Zepelin), amlexanox (Elics), pemirolast potassium (Alegysal), pemirolast potassium (Pemilaston), cromolyn sodium (Intal), tranilast (Rizaben), tranilast (Tramelas), ibudilast (Ketas), β2-adrenergic agonists, cromolyn sodium, cromolyn, ketotifen, methylxanthine, omalizumab, pemirolast, and quercetin, and the mast cell stabilizer is preferably cromolyn, pemirolast or a salt thereof.

### <Chemical mediator release inhibitor>

A chemical mediator release inhibitor is a drug that suppresses the release of chemical mediators from immune cells, such as mast cells, to suppress allergic reactions, such as a drug that stabilizes the cell membrane of mast cells to suppress the release of histamine, or the like, to the outside, such as a drug that inhibits the release of, from mast cells, allergens (substances that cause allergic reactions) such as LTB4, LTC4, LTD4, PGD2, TXB2, and PAF. Examples of chemical mediator release inhibitors include acitazanolast hydrate solution (Zepelin), amlexanox (Elics), pemirolast potassium (Alegysal), pemirolast potassium (Pemilaston), cromolyn sodium (Intal), tranilast (Rizaben), tranilast (Tramelas), ibudilast (Ketas), β2-adrenergic agonists, cromolyn sodium, cromolyn, ketotifen, methylxanthine, omalizumab, pemirolast, and quercetin, and are preferably cromolyn or pemirolast or a salt thereof.

### <Lactic acid bacteria>

Examples of lactic acid bacteria include homolactic acid bacteria and heterolactic acid bacteria. Additional examples of lactic acid bacteria include spherical lactic acid cocci and rod-shaped lactic acid bacillus. Examples of lactic acid bacteria include Gram-positive, bacillus or cocci, non-spore-forming, non-motile fungi that produce 50% or more of lactic acid relative to consumed glucose, and that require niacin (B3). Examples of lactic acid bacteria include intestinal lactic acid bacteria, animal lactic acid bacteria, vegetable lactic acid bacteria, and marine lactic acid bacteria. Examples of lactic acid bacteria include lactic acid bacteria of the order Lactobacillales and phylum Actinomycetes. Examples of lactic acid bacteria of the order Lactobacillales include lactic acid bacteria of the genus Lactobacillus, lactic acid bacteria of the genus Enterococcus, lactic acid bacteria of the genus Lactococcus, lactic acid bacteria of the genus Pediococcus, lactic acid bacteria of the genus Leuconostoc, and lactic acid bacteria of the genus Streptococcus. Examples of lactic acid bacteria in the Actinomycetes phylum include lactic acid bacteria in the genus Bifidobacterium. Examples of lactic acid bacteria of the genus Lactobacillus include Lactobacillus paracasei, Lactobacillus plantarum, Lactobacillus brevis, Lactobacillus delbrueckii, Lactobacillus acidophilus, Lactobacillus casei, Lactobacillus bulgaricus, Lactobacillus gasseri, Lactobacillus acidophilus, Lactobacillus fructivorans, Lactobacillus hilgardii, Lactobacillus rhamnosus, Lactobacillus plantarum, and Lactobacillus casei Shirota. Examples of lactic acid bacteria of the genus Enterococcus include Enterococcus faecalis and Enterococcus faecium. Examples of lactic acid bacteria of the genus Lactococcus include Lactococcus lactis and Lactococcus cremoris. Examples of lactic acid bacteria of the genus Pediococcus include Pediococcus damnosus. Examples of lactic acid bacteria of the genus Leuconostoc include Leuconostoc mesenteroides. Examples of lactic acid bacteria of the genus Streptococcus include Streptococcus thermophiles and Streptococcus mutans. Examples of lactic acid bacteria of the genus Bifidobacterium include Bifidobacterium bifidum and Bifidobacterium adolescentis.

It is known (Sohn et al, Dig Dis Sci, 2015 Nov;60(11): 3340-50) that administration of Lactobacillus paracasei shifts the Kupffer cells (liver-resident macrophages) in the liver to M2, thereby improving symptoms of non-alcoholic steatohepatitis. It is known (Morita et al, Nutrients, 2018 Dec15;10(12): 1991) that administration of Lactobacillus paracasei activates M2 macrophages, thereby mitigating blue-light-induced retinal degeneration. It is known (Jang et al, Int Immunopharmacol, 2014 Jul;21(1): 186-92) that administration of Lactobacillus plantarum promotes polarization of M1 macrophages to M2 macrophages, thereby alleviating colitis. It is known (Jang et al, J Appl Microbiol, 2013 Sep;115(3):888-96) that administration of Lactobacillus brevis promotes polarization of M1 macrophages to M2 macrophages, thereby alleviating colitis. In this manner, it is known that administration of lactic acid bacteria of the order Lactobacillus, such as the genus Lactobacillus, induces and activates M2 macrophage polarization. Therefore, the lactic acid bacteria are preferably lactic acid bacteria of the order Lactobacillus, such as the genus Lactobacillus.

A drug delivery system (DDS) may be used for the delivery, to the subject's choroid, of an M2 macrophage polarization inducer or a choroid-resident immune cell function regulator, such as a mast cell stabilizer or a chemical mediator release inhibitor.

For example, when the M2 macrophage polarization inducer or a choroid-resident immune cell function regulator, such as a mast cell stabilizer or a chemical mediator release inhibitor, is a nucleic acid, DNA or RNA (messenger RNA) that encodes the M2 macrophage polarization inducer or the choroid-resident immune cell function regulator, such as a mast cell stabilizer or a chemical mediator release inhibitor, may be administered to the subject using a DDS, such as liposome. Other DDSs that can be used include charged lipids, nucleic acid-protein complexes, and biopolymers. In addition, an M2 macrophage polarization inducer or a choroid-resident immune cell function regulator, such as a mast cell stabilizer or a chemical mediator release inhibitor, may be administered to the subject in the form of protein. Administration is performed, for example, by local administration to the choroid.

The delivery of an M2 macrophage polarization inducer or a choroid-resident immune cell function regulator, such as a mast cell stabilizer or a chemical mediator release inhibitor, to the subject may be performed by transplanting, into the subject, cells that secrete the M2 macrophage polarization inducer or choroid-resident immune cell function regulator, such as a mast cell stabilizer or a chemical mediator. Cells that secrete an M2 macrophage polarization inducer or a choroid-resident immune cell function regulator, such as a mast cell stabilizer or a chemical mediator, may be, for example, retinal pigment epithelial cells (RPE). Alternatively, the cells may have been genetically engineered to secrete an M2 macrophage polarization inducer or a choroid-resident immune cell function regulator, such as a mast cell stabilizer or a chemical mediator.

Subjects of the method of inhibiting or treating myopia of the present disclosure may be mammals including humans, or non-human mammals including dogs, cats, cows, and horses, but are preferably humans.

### [Composition for inhibiting or treating myopia]

One aspect of the present disclosure relates to a composition for inhibiting, improving, or treating myopia using an M2 macrophage polarization inducer, a choroid-resident immune cell function regulator, such as a mast cell stabilizer or a chemical mediator release inhibitor, or lactic acid bacteria. More specifically, one embodiment of the present disclosure relates to a composition for inhibiting or treating myopia, the composition comprising a therapeutically effective amount of an M2 macrophage polarization inducer, a choroid-resident immune cell function regulator such as a mast cell stabilizer or a chemical mediator release inhibitor, or lactic acid bacteria. Such a composition can be used to deliver, to the subject's choroid, the M2 macrophage polarization inducer, the choroid-resident immune cell function regulator, such as a mast cell stabilizer or a chemical mediator release inhibitor, or the lactic acid bacteria. Such a composition may be a pharmaceutical composition, a food product, or a supplement.

A pharmaceutical composition according to the present disclosure is, for example, administered locally to the eye. Examples of dosage forms of the present composition include ocular instillation (including application of eye ointment and eye washing), subconjunctival administration, administration into the conjunctival sac, and sub-Tenon's administration.

The dosage form of the present composition is not particularly limited, but includes, for example, eye drops, eye ointments, injections, patches, gels, inserts, and the like, with eye drops being preferred. The dosage forms described above can be prepared using ordinary techniques commonly used in the relevant field.

Eye drops can be prepared using an agent selected as necessary from among isotonic agents such as sodium chloride, potassium chloride, and concentrated glycerin; buffering agents such as sodium phosphate, sodium acetate, and epsilon-aminocaproic acid; surfactants such as polyoxyethylene sorbitan monooleate, polyoxyl 40 stearate, and polyoxyethylene hydrogenated castor oil; stabilizers such as sodium citrate and sodium edetate; and preservatives such as parabens. The pH may be within a range acceptable for ophthalmic formulations, but is preferably within the range of 4 to 8 under normal conditions. Eye ointments can be prepared using commonly-used bases such as white petrolatum and liquid paraffin.

In addition, the pharmaceutical composition according to the present disclosure is not limited to local administration to the eye, and may be administered via any administration route, such as enteral administration (oral, tubal, intramuscular, etc.) or parenteral administration (intravenous, intraarterial, transdermal, intramuscular, etc.). The dosage form of the composition used for these administration forms can be selected, as appropriate, and can be, for example, solid preparations such as tablets, granules, powders, capsules, or chewable tablets, or liquid preparations such as solutions, syrups, injections, or drips.

The pharmaceutical composition according to the present disclosure may contain appropriate amounts of other components within the range of not impairing the effects of the present invention. Examples of other components include any carrier, buffer, diluent, excipient, suspending agent, lubricant, adjuvant, vehicle, delivery system, emulsifier, disintegrant, absorbent, preservative, surfactant, colorant, flavoring agent, or sweetener. These components may be mixed individually or in appropriate combinations of two or more.

The content proportion of the M2 macrophage polarization inducer, the choroid-resident immune cell function regulator, such as a mast cell stabilizer or a chemical mediator release inhibitor, or the lactic acid bacteria in 100% by weight of the pharmaceutical composition can be set, as appropriate, within the range of 0.001 to 99.99% by weight, for example.

The dosage of the pharmaceutical composition according to the present disclosure is not particularly limited, and may be selected, as appropriate, in accordance with the administration form, the age and weight of the administration subject, the degree of the desired effect, and the like. The dosage of the M2 macrophage polarization inducer, the choroid-resident immune cell function regulator, such as a mast cell stabilizer or a chemical mediator release inhibitor, or the lactic acid bacteria is, for example, 100 - 1,000,000 nmol, and preferably 150 - 100,000 nmol, per day, and the administration frequency can be, for example, 1 to 100 times per month.

In addition, the composition according to the present disclosure may be a food product or a supplement. The form of the food product or supplement may be, for example, liquid, solid, tablet, granule, powder, capsule, paste, gel, etc., and may be selected from the solid or liquid formulations described above, for example. Specific examples of food products include various general processed foods, such as fruit juice drinks, vegetable juice, soft drinks, tea, and other beverages, soup, pudding, yogurt, premixed cake products, confectioneries, cookies, candies, gummies, and chewing gum, as well as special purpose foods, foods for specified health uses, foods with nutrient function claims, functional foods, nutritional supplements, health supplements, nutritionally fortified foods, and nutritionally adjusted foods, such as supplements and energy drinks.

The food products or supplements according to the present disclosure may contain any functional ingredients (vitamins, minerals, etc.), any excipients, and any additives (flavoring agents, sweeteners, acidulants, colorants, thickeners, binders, strengthening agents, disintegrants, buffers, surfactants, solubilizers, resorption promoters, dispersants, stabilizers, gelling agents, emulsifiers, antioxidants, surfactants, preservatives, desiccants, pH adjusters, colorants, soothing agents, tonicity agents, etc.).

One aspect of the present disclosure relates to the use of an M2 macrophage polarization inducer, a choroid-resident immune cell function regulator, such as a mast cell stabilizer or a chemical mediator release inhibitor, or lactic acid bacteria, for the purpose of inhibiting or treating myopia. Furthermore, another aspect of the present disclosure relates to the use of an M2 macrophage polarization inducer, a choroid-resident immune cell function regulator, such as a mast cell stabilizer or a chemical mediator release inhibitor, or lactic acid bacteria, in the manufacture of a pharmaceutical used for inhibiting or treating myopia.

### [Screening method]

One aspect of the present disclosure also relates to a screening method for searching for a component that promotes polarization of macrophages into M2 macrophages in order to control choroidal thinning, which is the mechanism of myopia pathogenesis, and a screening method for searching for a component that is effective in the prevention and treatment of myopia.

In some embodiments, the screening method comprises (i) a step for administering a candidate substance to an animal model, and (ii) a step for measuring axial length, choroidal thickness, and refractivity in the animal model. In some embodiments, the screening method comprises (i) a step for administering a candidate substance to an animal model, and (ii) a step for measuring the expression of a choroid-resident immune cells marker, such as an M2 macrophage marker, in a sample taken from the animal model. In some embodiments, the screening method further comprises (iii) a step for measuring the expression of a choroid-resident immune cell marker, such as an M1 macrophage marker.

In addition, some embodiments include an in vitro screening method in which cells, such as choroid-resident immune cells, such as monocytic cell lines, e.g., RAW264 cells, J774 cells, and U937 cells, mouse peritoneal macrophages, and bone marrow-derived macrophages are cultured in a medium containing the candidate substance, and the expression of a choroid-resident immune cell marker such as an M2 macrophage marker is evaluated.

In some embodiments, the animal model may be an animal undergoing a myopia-inducing procedure.

In some embodiments, the choroid-resident immune cell marker, such as an M2 macrophage marker, may be CD163, CD206, arginase, or IL-10. Methods of measuring the choroid-resident immune cell markers, such as macrophage markers, include quantitative PCR, flow cytometry, immunostaining, luciferase assay, and arginase activity staining.

Substances identified by means of such a screening method can be used for inhibiting or treating myopia.

While preferred embodiments of the present invention are illustrated in the present Specification, it would be apparent to a person skilled in the art that such embodiments are provided only for illustrative purposes, and that various modifications, changes, and substitutions may be made by the skilled person without departing from the present invention. It should be understood that various alternative embodiments of the invention described in the present Specification may be used when implementing the present invention. Furthermore, the present application claims the benefit of priority from Japanese Patent Application No. 2022-72596. The content of said Japanese Patent Application, as well as the content described in all publications, including patents and patent application documents referenced in the present Specification, should be construed as incorporated herein by reference to the same extent as if the content were explicitly recited in this Specification.

### Examples

The present invention will be described in further detail with reference to experimental examples.

### <Test Example 1: Choroidal thinning and myopization induced by LPS administration>

In order to confirm whether M1 macrophage polarization is involved in choroidal thinning and myopization, choroidal thickness, axial length, refractive value, and the expression of polarization marker genes in the choroid after LPS administration were measured.

The choroidal thickness, axial length, and refractive value were measured in mice administered LPS for two weeks and in control mice administered PBS, and the amounts of changes therein were calculated (Figures 1A and B). The eyes were then enucleated and the expression of polarization marker genes was measured using quantitative PCR (Figure 2).

C57BL6J mice (n=4 per group) were used.

The LPS administration group was administered LPS solution intraperitoneally daily at a dose of 10 mg/kg BW.

The PBS administration group was administered PBS instead of LPS solution.

### (Measurement of axial length, choroidal thickness, and refractivity)

The axial length, choroidal thickness, and refractivity of the mice in each group were measured. The axial length and the choroidal thickness were measured using spectral domain optical coherence tomography (Envisu R4310, manufactured by Leica). The refractivity was measured using an infrared photorefractor for mice (made by Professor Schaeffel, University of Tubingen).

### (Observation of choroid using an electron microscope)

Eyes from mice in each group were harvested and fixed overnight at 4°C in 2.5% glutaraldehyde in phosphate buffered saline (PBS), then rinsed for 1 hour with 0.1M sodium cacodylate buffer. Subsequently, the eyes were fixed in 1% OsO4 in 0.1M cacodylate buffer for 2 hours, followed by dehydration through a graded series of ethanol solutions. Furthermore, the eyes were infiltrated overnight in a 1:2 mixture of propylene oxide and Epon-Araldite, then embedded in 100% resin. The blocks were sectioned and observed using a transmission electron microscope (JEM1400 plus; JEOL) at an accelerating voltage of 100 kV.

### (Evaluation of gene marker expression)

As described above, samples of the choroid, retina, and sclera were collected from mice administered LPS for two weeks, and the expression of M1 macrophage marker genes and oxidative-stress-related genes was analyzed using the quantitative PCR method.

### (Results)

Compared to the control group that was administered PBS, the LPS administration group displayed refractive loss (Figure 1B, left), ocular elongation (Figure 1B, center), and choroidal thinning (Figure 1B, right) in both the one-week and two-week administration periods.

In addition, the gene expression analysis revealed increased expression of M1 marker genes and oxidative-stress-related genes, particularly in the choroid (Figure 2).

These results confirmed that an increase in M1 macrophages in the choroid induces myopization.

### <Test Example 2: M2 macrophage polarization and myopia suppression effect by means of IL-4 and IL-13 administration>

M1 macrophages are mainly involved in the induction of inflammation, whereas M2 macrophages play an antagonistic role, involved in the resolution and suppression of inflammation. Since M1 macrophages have a myopia-inducing effect, M2 macrophages are thought to have an inhibitory effect on myopia and the progression thereof.

Therefore, IL-4, which is a cytokine necessary for M2 macrophage polarization, was administered to mice, and whether M2 polarization would occur in the choroid was evaluated by means of analyzing the expression levels of M2 macrophage markers CD163 and CD206 (Figures 3A and B).

### <IL-4 administration>

C57BL6J mice (n=4 per group) were intraperitoneally administered 0.1 µg/100 µl (10 µg/kg BW) of IL-4 solution. Choroid, retina, and liver samples were taken at 0 hours (before administration) as well as 4, 24, and 48 hours after administration, and the expression of M2 macrophage marker genes was analyzed by Western blot and real-time PCR (Figure 3). In addition, choroid and retina samples were collected at 0 hours (before administration) as well as 4 and 24 hours after administration, and the expression of oxidative-stress-related genes was analyzed by real-time PCR (Figure 4).

Additionally, IL-4 was administered to mice undergoing myopia induction to evaluate whether there is a myopia suppression effect (Figure 4).

C57BL6 mice undergoing myopia induction (-30D lens, n=4) were intraperitoneally administered 0.1 µg/100 µl (10 µg/kg BW) of IL-4 solution for 3 weeks. C57BL6J mice undergoing myopia induction (0D lens, -30D lens, n=4 per group) were used as control groups. After 3 weeks, refraction, ocular elongation, and choroidal thinning were measured in the same manner as in Test Example 1.

### (Results)

As a result, IL-4 administration increased the expression of M2 markers CD163 and CD206 in the choroid (Figure 3B and C), and M2 macrophage polarization was confirmed. In addition, the gene expression analysis revealed increased expression of M2 macrophage marker genes and suppressed expression of oxidative-stress-related genes (Figure 5). These results suggest that IL-4 administration induces M2 macrophage polarization, suppresses the expression of oxidative-stress-related genes, and inhibits the progression of myopia (Figure 5).

Furthermore, it was confirmed that administration of IL-4 during the myopia induction period suppresses refractive loss (Figure 5B, left), ocular elongation (Figure 5B, center), and choroidal thinning (Figure 5B, right).

These results confirmed that M2 polarization of choroidal macrophages by means of IL-4 administration has a myopia suppression effect.

### <IL-13 administration>

IL-13, which is known as a cytokine that causes M2 polarization of macrophages, in the same manner as IL-4, was administered during the myopia induction period, and it was confirmed, using C57BL6J mice, that minus-lens-induced ocular elongation, myopic shift in refraction, and choroidal thinning are suppressed, and that M2 macrophage polarization is induced as a result (Figure 6).

Changes in blood flow and the number of macrophages were measured as follows.

Changes in blood flow were measured using swept-source optical coherence tomography (OCT S-1, Canon).

The number of macrophages was measured using a flow cytometer (CytoFLEX S, Beckman Coulter) after digestion of the choroid and staining with F4/80 antibody and CD11b antibody. The number of M2 macrophages was measured in the same manner as for the measurement of the number of macrophages, except for the addition of a staining process with CD206 antibody.

As can be seen in the results of Test Examples 1 and 2, it was confirmed that it is possible to induce or inhibit myopia by controlling the state of macrophages, particularly in the choroid. From the viewpoint of treatment and inhibition of progression of myopia, the foregoing indicates that promoting M2 macrophage polarization is particularly effective in achieving said objectives.

### <Test Example 3: Suppression of myopization by means of mast cell stabilizer instillation>

To verify the inhibitory effect of mast cell stabilizer instillation on myopia progression, a lens-induced myopia model was employed, wherein myopia was induced in mice by fitting the mice with minus lenses. During the myopia induction period, either cromolyn solution (4% solution) or pemirolast potassium (0.1% solution) was administered once daily by means of ocular instillation.

Following the test method described above, the axial length, refractive value, and choroidal thickness were measured in mice that underwent myopia induction for 3 weeks and ocular instillation of cromolyn solution or pemirolast potassium solution, as well as in control mice, and the amounts of changes therein were calculated (Figures 7A, B, and C).

As a result, the eyes fitted with the minus lenses in the control group that had been administered PBS showed ocular elongation (Figure 7A), myopic shift in refraction (Figure 7B), and choroidal thinning (Figure 7C) compared with the control eyes. On the other hand, the above-mentioned changes observed in the PBS administration group were not observed in the groups administered with eye drops of cromolyn solution or pemirolast potassium solution.

These results demonstrate that ocular instillation of a mast cell stabilizer suppresses the progression of myopia.

### <Test Example 4: Comparison of myopia suppression effect between instillation of mast cell stabilizer and of histamine receptor antagonist>

Anti-allergic drugs are broadly divided into two types: mast cell stabilizers, which inhibit mast cell degranulation, and histamine receptor inhibitors, which inhibit the action of histamine secreted due to mast cell degranulation. In order to clarify whether the myopia suppression effect of the mast cell stabilizer instillation in Test Example 3 was due to the action of the anti-allergic drug or to the inhibition of mast cell degranulation, pemirolast potassium (0.1% solution) and levocabastine solution (0.025% solution) were administered as eye drops, respectively as a mast cell stabilizer and a histamine receptor inhibitor, to mice with LIM-induced myopia, and the myopia-suppressing effects were compared. The results are shown in Figure 8.

As shown in Figure 8, the results were similar to those of Test Example 3, and the ocular elongation, myopic shift in refraction, and choroidal thinning observed in the control group were not observed in the pemirolast potassium administration group. On the other hand, ocular elongation, myopic shift in refraction, and choroidal thinning were observed in the levocabastine administration group, similar to the control group.

From the results described above, it was confirmed that mast cell stabilizers, among other anti-allergy drugs, have a myopia suppression effect.

### <Test Example 5: Inhibition of myopia progression by means of Lactobacillus paracasei administration>

Since induction of M2 macrophages can suppress the progression of myopia, Lactobacillus paracasei, a lactic acid bacterium contained in Yakult (Yakult Honsha Co., Ltd.), was cultured, grown, and administered to myopic model mice. As in Test Examples 3 and 4, the axial length, refractive value, and choroidal thickness were measured and the amounts of changes therein were calculated (Figure 9). As a result, it was confirmed that administration of lactic acid bacteria suppresses the progression of myopia (Figure 9).

## Claims

1. A method for inhibiting, improving, or treating myopia, comprising administering to a subject in need thereof a therapeutically effective amount of a choroid-resident immune cell function regulator or lactic acid bacteria.

2. The method according to claim 1, wherein the choroid-resident immune cell function regulator is an M2 macrophage polarization inducer, a mast cell stabilizer, or a chemical mediator release inhibitor.

3. The method according to claim 2, wherein the M2 macrophage polarization inducer is selected from the group consisting of: proteins, peptides, nucleic acids, small molecule compounds, and polymeric compounds.

4. The method according to claim 2, wherein the M2 macrophage polarization inducer is selected from the group consisting of: IL-4, IL-10, IL-13, TLR2, 4, 7, 9 and ligands thereof, bisantrene dihydrochloride, triptolide, lovastatin, QS11, regorafenib, sorafenib, ixazomib, GW-843682X, KW 2449, axitinib, JTE 013, purmorphamine, arcyriaflavin A, dasatinib, NVP-LDE225, 1-naphthyl PP1, MGCD-265, and bosutinib.

5. The method according to claim 2, wherein the mast cell stabilizer or the chemical mediator release inhibitor is cromolyn, pemirolast or a salt thereof.

6. The method according to claim 1, wherein the lactic acid bacteria is of the genus Lactobacillus.

7. The method according to claim 1, wherein the lactic acid bacteria is Lactobacillus paracasei.

8. The method according to claim 1, wherein the choroid-resident immune cell function regulator or the lactic acid bacteria is included in a pharmaceutical composition, a supplement, or a food product.

9. The method according to claim 1, wherein refractive loss, ocular elongation, and/or choroidal thinning is inhibited.

10. A composition for inhibiting or treating myopia, the composition comprising a therapeutically effective amount of a choroid-resident immune cell function regulator or lactic acid bacteria.

11. A supplement for inhibiting or improving myopia, the supplement comprising an effective amount of a choroid-resident immune cell function regulator or lactic acid bacteria.

12. A food product for inhibiting or improving myopia, the food product comprising an effective amount of a choroid-resident immune cell function regulator or lactic acid bacteria.

13. Use of a choroid-resident immune cell function regulator or lactic acid bacteria in manufacture of a pharmaceutical for inhibiting or treating myopia.

14. Use of a choroid-resident immune cell function regulator or lactic acid bacteria for inhibiting or treating myopia.
